# EUROPEAN PATENT APPLICATION

(11) **EP 1 872 778 A1**
(43) Date of publication of application: **02.01.2008**
(21) Application number: 06013440.0
(22) Date of filing: 29.06.2006
(51) Int. Cl.: A61K 9/36, A61K 9/32, A61K 31/4439

(54) **Stable pharmaceutical composition with rabeprazole sodium**

(71) Applicant: LEK Pharmaceuticals d.d., 1526 Ljubljana (SI)
(72) Inventor: The designation of the inventor has not yet been filed
(74) Representative: Dietz, Jörg-Reimar

(57) **Abstract**

The present invention relates to pharmaceutical composition containing rabeprazole or a pharmaceutically acceptable salt thereof especially sodium salt of rabeprazole. The subject of the invention is a stable pharmaceutical composition comprising rabeprazole sodium, calcium hydroxide, at least one pharmaceutically acceptable excipient and a separating layer between a tablet core and acidic coating of a tablet.

## Description

### Field of the invention

The present invention relates to pharmaceutical composition containing rabeprazole or a pharmaceutically acceptable salt thereof especially sodium salt of rabeprazole.

### Background of the invention

Rabeprazole is disclosed in European patent EP 268956 and there is presented its proton pump inhibition activity. Therein are also disclosed a synthesis and formulation of rabeprazole into a pharmaceutical composition. Rabeprazole is used for treatment or prevention of peptic ulcers.

Stability of rabeprazole sodium itself is poor. In particular, it is rapidly decomposed and colored under moist conditions or in an acidic to neutral aqueous solution. When rabeprazole sodium is to be formulated into a preparation for oral administration, therefore, they should be coated with an enteric coating to thereby prevent the decomposition of the same with gastric acid. However an enteric coating is an acidic material which is insoluble in water under acidic conditions and soluble in water under neutral to alkaline conditions. Thus the coating of a core comprising an acid-unstable compound might generally cause the decomposition of said acid-unstable compound. Such decomposition occurs even during the enteric coating stage by a common method, for example, with the use of a fluidized bed coater, which results in the coloration of the surface of the core. Further the storage stability of the coated core as well as the stability in an acidic solution of the same might be lowered thereby.
In order to avoid these difficulties, Japanese Patent Laid-Open No. 258316/1987 and No. 258320/1987 each disclose a method comprising intermediately coating the core containing an acid-unstable compound with a material soluble in water or decomposable in water and then further coating the same with an enteric coating. However these methods cannot sufficiently stabilize an acid-unstable compound and therefore further improvement is required.
An European patent application EP 1004305 disclose a composition of rabeprazole sodium with specially selected excipient such as potassium carbonate, sodium hydroxide, potassium hydroxide and other fillers such as aminoalkyl methaacrylate copolymer E. Those fillers are found to be compatible with rabeprazole sodium but for example sodium hydroxide is very hygroscopic compound and therefore could cause problems with stability of rabeprazole as it binds water into the formulation.

Other well known excipient for the stabilization of rabeprazole or its sodium salt is also magnesium oxide. Magnesium oxide is frequently used, since it also acts as insoluble filler. It is very slightly soluble in pure water and the saturated solution yields a pH of about 10. Its main disatvatage is that it is very hygroscopic, which is not beneficial in rabeprazole tablet production since rabeprazole is extremley water sensible.

As can be concluded from all said stable pharmaceutical composition of rabeprazole is desired and in the present invention this problem is solved.

### Summary of the invention

The subject of the invention is a pharmaceutical composition comprising rabeprazole sodium, calcium hydroxide, at least one pharmaceutically acceptable excipient and a separating layer between a tablet core and acidic coating of a tablet.

The pharmaceutical composition of a present invention can additionally contains pharmaceutically acceptable excipient such as of non-alkaline fillers, diluents, binders, disintegrants, flow conditioners, antiadherents and lubricants.

The pharmaceutical composition of a present invention contains a separating layer wherein separating layer can consists of one of excipients, selected from a group of polyvinylpyrrolidone, hypromellose and neutral polymethacrylates.

Another aspect of the invention is the use of the composition of a present invention in a treatment or prevention of peptic ulcers.

### Detailed description of the invention

Rabeprazole sodium is an active compound from a group of proton pump inhibitors, which are known to be unstable in neutral and acidic media.

For the preparation of the appropriate composition we performed an investigation on compatibility of the rabeprazole sodium with various excipients in order to select the optimal combination for the final dosage form. We performed the investigation of increase of related substances and degradation products for selected excipient in the formulation after 7 days at 60°C and measured the said increase with HPLC chromatography. The results are presented in the Table 1.

**Table 1: Compatibility study results: increase in related substances and degradation products, after 7 days at 60°C:**

| Ingredient | Increase in related substances and degradation products in % |
|---|---|
| meglumine | 0,20 |
| sodium hydroxide | 0,45 |
| magnesium oxide | 0,27 |
| calcium hydroxide | 0,04 |
| L-arginine | 0,14 |
| anydrous lactose | 12,06 |
| lactose monohydrate | 18,26 |
| sodium stearyl fumarate | 0,18 |

As we surprisingly found the best results were achieved with calcium hydroxide. It is very important to have even distribution of calcium hydroxide because it assures that the tablet core of the pharmaceutical composition is evenly alkalized and this fact contributes the most to the stability of the formulation of the present invention. Another reason for a good stability of formulation in the present invention is the fact that calcium hydroxide is not hygroscopic. Therefore, it does not adhere water and in such manner further stabilizes rabeprazole sodium in the composition.

Solution of the present invention is therefore to prepare pharmaceutical formulation with rabeprazole sodium, calcium hydroxide as an alkalizing agent and a compatible exipients. Pharmaceutical formulation of the present invention is in the form of tablets. Pharmaceutical formulation has an enteric coating in order to protect the agent form the acidic gastric milieu. After the tablet has passed through the duodenum and jejunum the gastric coating dissolves and the agent begins to dissolve from the formulation.

Additionally in the pharmaceutical formulation of the present invention a separating layer is needed in order for rabeprazole sodium to be stable within the tablet core. Therefore, a separating layer between tablet core comprising rabeprazole sodium and acidic coating of a tablet is deployed in order to prevent interactions between the acid-sensitive rabeprazole sodium and said acidic coating.

Higher pH values in the tablet core which are obtained by addition of calcium hydroxide provide a better stabilisation of rabeprazole sodium, whereas the partial solubility ensures a better distribution of the alkalizing agent over the components of the tablet core. An additonal advantage of calcium hydroxyde is that it is safe to handle with, especially its dispersion in water. The pharmaceutical composition allows a technically feasible process of creating a formulation with suitable biopharmaceutical properties.

Pharmaceutical composition of the present invention combines rabeprazole sodium, calcium hydroxide and non alkaline ingredients that are compatible with rabeprazole sodium. Said non-alkaline ingredients are selected from a group of mannitol, low substituted hydroxypropyl cellulose, sodium stearyl fumarate and magnesium stearate.

Pharmaceutical composition of the present invention can also comprise one or more fillers selected from a group of diluents, binders, disintegrants, flow conditioners, anti-adherents or lubricants.

Typically rabeprazole sodium will be present in a pharmaceutical composition of the present invention in an amount within the range of 5 to 50%, preferably 7 to 25%.

Calcium hydroxide will be present in a pharmaceutical composition of the present invention in an amount within the range of 0.1 % to 30%, preferably from 0.5% to 3%.

For the pharmaceutical composition of the present invention a separating layer is necessary to prevent interactions between the rabeprazole or its sodium salt, present in the core, and the acidic gastric coating. This separating layer can consists of one of the excipients, selected from a group of polyvinylpyrrolidone, hypromellose and neutral polymethacrylates.

The pharmaceutical composition of the present invention may be prepared using standard techniques and manufacturing processes generally known in the art.

Tablet cores, for example, may be prepared by wet granulation. The components (without alkalizing agent and lubricant) are blended, granulated with water or water-ethanol mixture with alkalizing agent suspended or dissolved in it. Wet granulate is dried and passed through a mesh screen to obtain uniform particle size of the granules. A lubricant is added to the dry granulate and blending continued until lubricated granulate is obtained. The mixture is then compressed into tablets. Alternatively, a direct compression technique, a well known technique from the art can be employed.

Onto the tablet cores the separating layer is deployed by spray-coating, with a water and/or ethanol based film coating formulation. Coating ingredient combinations are readily available and are usually fairly simple solutions of polymer in coating media.

Finally an enteric coating is applied using spray-coating techniques with water or organic based coating dispersions.

The composition of the present invention is illustrated by the following examples, which in no way limit the scope of the invention.

### EXAMPLE 1

### Preparation of the composition

Ingredients in the composition of the present invention are presented in the table 2 below:

**Table 2:**

| | ingredient | Mass percentage |
|---|---|---|
| tablet core: | | |
| | rabeprazole sodium | 11,5 |
| | calcium hydroxide | 0,6 |
| | mannitol | 56,0 |
| | low substituted hydroxypropyl cellulose | 17,2 |
| | sodium stearyl fumarate | 0,9 |
| separating layer | | |
| | polyvinylpyrrolidone | 1,7 |
| acid gastric coat | | |
| | hypromellose phthalate | 10,3 |
| | dibutylsebacate | 1,1 |
| | yellow pigment | 0,1 |
| | titanium dioxide | 0,5 |

### Technological procedure:

Technological procedure to obtain the pharmaceutical composition of the present invention is the following: rabeprazole sodium, mannitol and low substituted hydroxypropyl cellulose are mixed and granulated with water dispersion of calcium hydroxide in a high shear granulator. Wet granulate is dried and passed through a mesh screen to obtain uniform particle size of the granules. Sodium stearyl fumarate is added to the dry granulate and mixed for short period of time. The mixture is then compressed into tablets using rotary tablet machine. Tablet weight is 150 mg. Povidone in dissolved in ethanol and applied onto tablet cores using spray coating in perforated drums.
For a gastric acid coat hypromellose phthalate, dibutylsebacate and pigments are dissolved/dispersed in ethanol-acetone (1:1) mixture. Obtained coating mixture is applied onto tablet using spray coating in perforated drums.

### EXAMPLE 2

### Preparation of the composition

Ingredients in the composition of the present invention are presented in the table 3 below:

**Table 3:**

| | ingredient | Mass percentage |
|---|---|---|
| tablet core: | | |
| | rabeprazole sodium | 11,5 |
| | calcium hydroxide | 3,0 |
| | mannitol | 45,1 |
| | low substituted hydroxypropyl cellulose | 25,8 |
| | magnesium stearate | 0,9 |
| separating layer | | |
| | hypromellose | 1,4 |
| | talc | 0,3 |
| acid gastric coat | | |
| | hypromellose phthalate | 10,3 |
| | dibutylsebacate | 1,1 |
| | yellow pigment | 0,1 |
| | titanium dioxide | 0,5 |

### Technological procedure:

Technological procedure to obtain the pharmaceutical composition of the present invention is the following: rabeprazole sodium, mannitol and low substituted hydroxypropyl cellulose are mixed and granulated with water dispersion of calcium hydroxide in a high shear granulator. Wet granulate is dried and passed through a mesh screen to obtain uniform particle size of the granules. Magnesium fumarate is added to the dry granulate and mixed for short period of time. The mixture is then compressed into tablets using rotary tablet machine. Tablet weight is 150 mg. Hypromellose is dissolved and talc is dispersed in water/ethanol mixture (1:9). Obtained dispersion is applied onto tablet cores using spray coating in perforated drums.
For a gastric acid coat hypromellose phthalate, dibutylsebacate and pigments are dissolved /dispersed in ethanol-acetone (1:1) mixture. Obtained coating mixture is applied onto tablet using spray coating in perforated drums.

## Claims

1. A pharmaceutical composition comprising rabeprazole sodium, calcium hydroxide, at least one pharmaceutically acceptable excipient and a separating layer between a tablet core and acidic coating of a tablet.

2. The pharmaceutical composition according to claim 1 wherein pharmaceutically acceptable excipient is selected from a group of non-alkaline fillers, diluents, binders, disintegrants, flow conditioners, antiadherents and lubricants.

3. The pharmaceutical composition according to claim 2 wherein non-alkaline fillers are selected from a group of of mannitol, low substituted hydroxypropyl cellulose, sodium stearyl fumarate and magnesium stearate.

4. The pharmaceutical composition according to claim 1 wherein the separating layer consisting of at least one of the excipients selected from a group of polyvinylpyrrolidone, hypromellose and neutral polymethacrylates.

5. The pharmaceutical composition according to claim 1 wherein said composition comprises about 5 to 50% of rabeprazole sodium.

6. The pharmaceutical composition according to claim 1 wherein said composition comprises about 0.3 to 25% of rabeprazole sodium.

7. The pharmaceutical composition according to claim 1 wherein said composition comprises about 0.1 to 30% of calcium hydroxide.

8. The pharmaceutical composition according to claim 1 wherein said composition comprises about 0.5 to 3% of calcium hydroxide.

9. The use of the composition according to any of claims 1 to 8 in a treatment or prevention of peptic ulcers.
